(19) Europäisches Patentamt
European Patent Office
Office européen des brevets

(11) Publication number: **0 482 417 A1**

(12) **EUROPEAN PATENT APPLICATION**

(21) Application number: **91117109.8**

(51) Int. Cl.[5]: **A61K 7/00**

(22) Date of filing: **08.10.91**

(30) Priority: **22.10.90 US 601966**

(43) Date of publication of application:
**29.04.92 Bulletin 92/18**

(84) Designated Contracting States:
**AT BE CH DE DK ES FR GB GR IT LI LU NL SE**

(71) Applicant: **THE B.F. GOODRICH COMPANY**
**3925 Embassy Parkway**
**Akron Ohio 44313-1799(US)**

(72) Inventor: **Castaneda, Janet Yvonne**
**4759 Meadow Rue**
**Toledo, Ohio 43614(US)**
Inventor: **Lochhead, Robert Yeats**
**305 Westever Drive, Apt. 16H**
**Hattiesburg, Mississippi 39402(US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Emulsion composition comprising a high molecular weight anionic emulsifying agent and a low molecular weight cationic surfactant, and methods relating thereto.**

(57) The present invention is directed to emulsion systems comprising an emulsifying agent having a weight average molecular weight in excess of 1000, wherein the emulsifying agent comprises a hydrophilic portion which is swellable and suspendable in water and a hydrophobic portion capable of directly or indirectly binding to a hydrophobic droplet; the hydrophilic portion comprises anionic functionality which enhances the hydrophilicity of the hydrophilic portion. The emulsion composition of the present invention is surprisingly stable even in the presence of cationic surfactant and is therefore particularly advantageous in conditioning lotions and the like, because cationic skin lotions are generally preferred commercially over nonionic or anionic lotions.

EP 0 482 417 A1

## FIELD OF THE INVENTION

The present invention is directed to emulsion systems comprising an emulsifying agent having a weight average molecular weight in excess of 1000, wherein the emulsifying agent comprises a hydrophilic portion which is swellable and suspendable in water and a hydrophobic portion capable of directly or indirectly binding to a hydrophobic droplet; the hydrophilic portion comprises anionic functionality which enhances the hydrophilicity of the hydrophilic portion. More specifically, the emulsion composition of the present invention is surprisingly stable even in the presence of up to about 5 weight percent cationic surfactant and is therefore particularly advantageous in personal care lotions and the like, because cationic lotions are generally preferred commercially over nonionic or anionic lotions.

The emulsifying agent structure of the present invention advantageously supports the emulsion substantially without neutralizing the cationic surfactant. Consequently, a much lower level of cationic surfactant is generally necessary relative to many conventional cationic emulsion systems, because the surfactant need not support the emulsion but instead need only be present in an amount sufficient to interact with the intended substrate, such as hair or skin, to thereby provide advantageous "skin feel" or "hair feel" benefits or the like. The present invention avoids an expensive and environmentally detrimental aspect of many conventional products-- extreme loadings of cationic surfactant.

## BACKGROUND OF THE INVENTION

Traditional skin lotions and creams generally comprise oil-in-water emulsions, and the emulsifiers are generally anionic or non-ionic surfactants. These emulsions are commonly stabilized (i.e., thickened) against creaming by the incorporation of anionic polyelectrolytes. Recently however, a new trend has come into existence wherein the emulsifying agent is a cationic, rather than anionic surfactant. Such cationic emulsions generally confer better skin or hair "feel" which is generally more durable than conventional anionic lotions.

Unfortunately however, cationic lotions generally require high loadings of cationic surfactant to achieve acceptable shelf stability. Such high loadings can cause skin irritation or the like. Furthermore, excessive concentrations of cationic surfactants can be environmentally harmful once the surfactant is washed from the skin. Indeed, only a relatively low loading of cationic surfactant is necessary to obtain the necessary "skin feel" or "hair feel" properties, and the excess surfactant is generally undesirable. The present invention is advantageous, because it allows for lower loadings of cationic surfactant, while maintaining the advantages of a cationic lotion system and while creating a stable, durable emulsion.

## SUMMARY OF THE INVENTION

The present invention is directed to emulsion systems comprising an emulsifying agent having a weight average molecular weight in excess of about 1000, wherein the emulsifying agent comprises a hydrophilic portion which is swellable and suspendable in water and a hydrophobic portion capable of directly or indirectly binding to a hydrophobic droplet; the hydrophilic portion comprises anionic functionality which enhances the hydrophilicity of the hydrophilic portion. The weight ratio of hydrophilic portion to hydrophobic portion of the emulsifying agent is preferably about 3-1000:1, more preferably about 5-100:1. The emulsion composition of the present invention is surprisingly stable even in the presence of up to about 5 weight percent cationic surfactant.

The emulsifying agent structure of the present invention advantageously supports the emulsion without neutralizing the cationic surfactant. Consequently, a much lower level of cationic surfactant is generally necessary relative to many conventional cationic emulsion systems, because the surfactant need not support the emulsion but instead need only be present in an amount sufficient to interact with the intended substrate, such as hair or skin. The present invention avoids an expensive and environmentally detrimental aspect of many conventional products-- extreme loadings of cationic surfactant.

## DETAILED DESCRIPTION OF THE PREFERRED EMBODIMENT

### Introduction

The present invention relates to oil-in-water emulsions comprising a high molecular weight emulsifying agent rather than a traditional low molecular weight surfactant. Whereas traditional surfactants are generally salts of fatty acids or derivatives thereof having a molecular weight generally much less than about 1000, the emulsifying agent of the present invention has a weight average molecular weight greater than about 1000, preferably about 50,000 to about 1,000,000.

The Emulsifying Agent

The high molecular weight emulsifying agent comprises a hydrophilic portion which is water swellable and water suspendable. This hydrophilic portion comprises anionic functionality. The weight ratio of hydrophilic portion to hydrophobic portion is preferably about 3-1000:1 and more preferably about 5-100:1.

Emulsifying Agent: Hydrophilic Portion

The hydrophilic portion can be any polymeric chain or network provided it is hydrophilic and provided it comprises anionic functionality and has a weight average molecular weight greater than about 1000. The preferred hydrophilic portion is a water insoluble, carboxylic-type polymeric material, such as polycarboxylic acid-type polymer or copolymer thereof, cross-linked or not. These hydrophilic polymer portions can be polymerized in water or in a solvent system other than water.

The preferred hydrophilic portion of the emulsifying agent comprises a carboxyl containing polymer having a weight average molecular weight greater than about 500 to several million, more preferably greater than about 10,000 to 1,000,000.

The carboxylic monomers useful in the production of the hydrophilic polymers portion of the emulsifying agent are the olefinically-unsaturated carboxylic acids containing at least one activated carbon-to-carbon olefinic double bond, and at least one carboxyl group. The acid contains at least one olefinic double bond which readily functions in a polymerization, due to its presence in the monomer molecule, either in the alpha-beta position with respect to a carboxyl group, i.e., -C=C-COOH, or as part of a terminal methylene grouping, i.e., $CH_2=C<$.

The presence of a methylene grouping in a carboxylic monomer makes this type of compound much more polymerizable than where the double bond is non-conjugated in the carbon structure. Olefinically-unsaturated acids of this class include such materials as the acrylic acids typified by the acrylic acid itself, methacrylic acid, ethacrylic acid, alpha-chloroacrylic acid, alpha-cyano acrylic acid, beta methylacrylic acid (crotonic acid), alpha phenyl acrylic acid, beta-acryloxy propionic acid, sorbic acid, alpha-chloro sorbic acid, beta-styryl acrylic acid (1-carboxy-4-phenyl butadiene-1,3), itaconic acid, citraconic acid, mesaconic acid, glutaconic acid, aconitic acid maleic acid, fumaric acid, tricarboxy ethylene, and the like. As used herein, the term "carboxylic acid" includes the polycarboxylic acids and those acid anhydrides, such as maleic anhydride, wherein the anhydride group is formed by the elimination of one molecule of water from two carboxyl groups located on the same polycarboxylic acid molecule. Acid anhydrides useful herein include those having one or more of the following functionalities:
halogen, cyanogen (-CN), alkyl, aryl, alkaryl, aralkyl, and cycloalkyl, such as methyl, ethyl propyl, octyl, decyl, phenyl, tolyl, xylyl, benzyl cyclohexyl, and the like.

The preferred carboxylic monomers for use in polymerizing the hydrophilic portion of the emulsifying agent for the present invention are the monoolefinic acrylic acids having the general structure

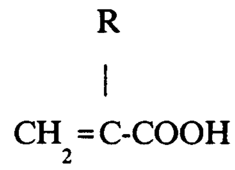

wherein R is a substituent selected from the class consisting of hydrogen, halogen, and the cyanogen groups, monovalent alkyl radicals, monovalent aryl radicals, monovalent aralkyl radicals, monovalent alkaryl radicals and monovalent cycloaliphatic radicals. Of this class, acrylic and methacrylic acid are most preferred due generally to lower cost, ready availability and an ability to form superior water swellable polymers. Another useful carboxylic monomer is maleic anhydride or the acid thereof.

The hydrophilic portion of the emulsifying agent contemplated include both homopolymeric carboxylic acids or anhydrides thereof, or the defined carboxylic acids copolymerized with one or more other vinylidene monomers containing at least one terminal $CH_2=CH<$ group. Depending upon the degree of hydrophilicity required in any particular embodiment of this invention, the hydrophilic portion may also include, for example, acrylic ester monomers including those acrylic ester monomers having long chain aliphatic groups such as derivatives of an acrylic acid represented by the formula

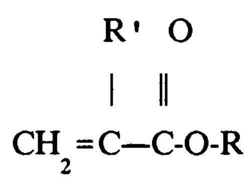

wherein R is an alkyl group having from 10 to 30 carbon atoms, preferably 10 to 20 carbon atoms and R' is hydrogen or a methyl or ethyl group, present in the copolymer in amount, for example, from about 1 to 30 weight percent, and for some uses more preferably, about 5 to 15 weight percent. Representative higher alkyl acrylic esters are

decyl acrylate, isodecyl methacrylate, lauryl acrylate, stearyl acrylate, biphenyl acrylate and melissyl acrylate and the corresponding methacrylates. Mixtures of two or three or more long chain acrylic esters may be successfully polymerized with one of the carboxylic monomers to provide a material useful in the present invention.

The hydrophilic portion of the emulsifying agent contemplated also include acrylamides and methacrylamides, particularly where the amide nitrogen is substituted with a $C_1$ -$C_{30}$ alkyl or aryl substituent to provide secondary and tertiary substituted acrylamides and methacrylamides. Useful acrylic amides include monoolefinically unsaturated amides which may be incorporated in the interpolymers of this invention have at least one hydrogen on the amide nitrogen and the olefinic unsaturation is alpha-beta to the carbonyl group. Representative amides include acrylamide, methacrylamide, N-methyl acrylamide, N-t-butyl acrylamide, N-cyclohexyl acrylamide, N-ethyl acrylamide and the like, such as $C_1$ - $C_{30}$ mono- and di-substituted acrylamides. Other acryl amides include N-alkylol amides of alpha, beta-olefinically unsaturated carboxylic acids including those having from 4 to 10 carbon atoms such as N-methylol acrylamide, n-ethanol acrylamide, n-propanol acrylamide, n-methylol methacrylamide, n-ethanol methacrylamide, N-methylol maleimide, N-methylol maleamide, N-methylol maleamic acid, N-methylol maleamide, N-methyl maleamic acid esters, the N-alkylol amides of the vinyl aromatic acids such as N-methylol-p-vinyl benzamide, and the like.

The hydrophilic portion of the emulsifying agent of the present invention may be cross-linked with any polyfunctional vinylidene monomer containing at least 2 terminal $CH_2 = C<$ groups, including for example, butadiene, isoprene, divinyl benzene, divinyl naphthalene, allyl acrylates and the like. A particularly useful cross-linking monomer is divinyl glycol. Other suitable cross linking agents include polyalkenyl polyethers having more than one alkenyl ether grouping per molecule. Preferred crosslinkers have alkenyl groups in which an olefinic double bond is present attached to a terminal methylene grouping, $CH_2 = C<$. They are made by etherification of a polyhydric alcohol containing at least 4 carbon atoms and at least 3 hydroxyl groups.

Compounds of this class may be produced by reacting an alkenyl halide, such as allyl chloride or allyl bromide with a strongly alkaline aqueous solution of one or more polyhydric alcohols. The product is a complex mixture of polyethers with varying numbers of ether groups. Efficiency of the polyether crosslinking agent increases with the number of potentially polymerizable groups on the molecule. It is preferred to utilize polyethers containing an average of two or more alkenyl ether groupings per molecule.

Other crosslinking monomers include for example, diallyl esters, dimethallyl ethers, allyl or methallyl acrylates and acrylamides, tetra-allyl tin, tetravinyl silane, polyalkenyl methanes, diacrylates and dimethacrylates, divinyl compounds as divinyl benzene, polyallyl phosphate, diallyloxy compounds and phosphite esters and the like. Typical agents are allyl pentaerythritol, allyl sucrose, trimethylolpropane triacrylate, 1,6-hexanediol diacrylate, pentaerythritol triacrylate, tetramethylene dimethacrylate, ethylene diacrylate, ethylene dimethacrylate, triethylene glycol dimethacrylate and the like. Other monomers can also be used, particularly in conjunction with acrylic esters, including the acrylic nitriles, $\alpha,\beta$-olefinically unsaturated nitriles useful in the interpolymers embodied herein are preferably the monoolefinically unsaturated nitriles having from 3 to 10 carbon atoms such as acrylonitrile, methacrylonitrile, ethacrylonitrile, chloroacrylonitrile, and the like. The monomer constituents may contain from 1-30 carbon atoms, including aliphatic, aromatic, and aralkyl; other functional groups may also be present, such as, $-SO_3H$, -COOH, $PO_3H_2$, ketone, and the like.

The amount of carboxyl containing monomer in the hydrophilic portion of the emulsifying agent is in the range of about 5% to about 100% by weight of monomer, preferably about 50%-100%. The non-carboxyl monomers preferably are vinylidene comonomers, such as alpha olefins containing from 2 to 12 carbon atoms, more preferably from 2 to 8 carbon atoms, such as ethylene, propylene, and the like. Suitable dienes include those containing from 4 to 10 carbon atoms, particularly conjugated dienes as butadiene, isoprene, piperylene and the like. Other useful comonomers include: vinyl esters and allyl esters, such as, vinyl acetate, vinyl benzoate, allyl acetate and the like; vinyl aromatics such as styrene, vinyl toluene, vinyl naphthalene and the like; vinyl and allyl ethers and ketones such as, vinyl methyl ether, ally methyl ether, vinyl isobutyl ether, and the like; vinyl nitriles such as acrylonitrile, methacrylonitrile and the like; cyanoalkyl acrylates, such as, alpha cyanomethyl acrylate and the like; vinyl halides and vinyl chloride and the like; halovinylates; and divinyls, diacrylates and other polyfunctional monomers, such as, divinyl benzene, divinyl ether, diethylene glycol diacrylate, ethylene glycol dimethacrylate and the like.

Emulsifying Agent: Hydrophobic Portion

The emulsifying agent of the present invention further comprises a hydrophobic portion. This hydrophobic portion extends away from the hydrophilic portion, thereby allowing the emulsifying agent to have a hydrophobic portion attracted or attached to a hydrophobic droplet and a hydrophilic portion suspended in water surrounding the droplet. More precisely, the hydrophobic portion, preferably pulls the emulsifying agent into the boundary layer region of the hydrophobic droplet, and provides direct or indirect attachment between the droplet and the emulsifying agent. In this way, the suspended emulsifying agent aids the droplet in remaining in suspension, because the water suspended hydrophilic portion helps maintain the droplet in suspension. Furthermore, the emulsifying agent substantially prevents flocculation, because the hydrophilic portions of the emulsifying agent for each droplet substantially repel one another.

The hydrophobic portion must be sufficiently hydrophobic to pull the emulsifying agent around each hydrophobic droplet, but not so hydrophobic as to hinder the hydrophilicity of the emulsifying agent. The emulsion comprises three phases-- the hydrophobic droplet phase; the hydrophilic continuous phase; and the hydrophobic-hydrophilic boundary layer phase therebetween. Preferably, the emulsifying agent is not so hydrophobic as to pull so much of the emulsifying agent into the droplet as to hinder the ability of the droplet to be suspended in the continuous phase. Also the emulsifying agent must not be so hydrophilic that the emulsifying agent will not enter the boundary layer phase and therefore not aid in suspending the droplet. Preferably, the hydrophobic portion will be quite small and will merely anchor the emulsifying agent to the droplet; the hydrophilic portion preferably is very large and preferably encompasses the boundary layer and extends into the continuous phase. Preferably, the boundary between the hydrophobic and hydrophilic portions of the emulsifying agent will be attracted to the hydrophilic-hydrophobic boundary layer.

Once the hydrophilic portion of the emulsifying agent is chosen as described above, the hydrophobic portion must be chosen, depending upon the hydrophobic droplet used in any particular system. One reasonably accurate method of predicting solubility is to determine the solubility parameter value of the material. Such values are available from Polymer Handbook, J. Brandsup and E. Immergut, editors, "Solvent Parameter Values" by H. Burrell, 1975, pp. IV-337-359 (hereafter "POLYMER HANDBOOK").

The preferred hydrophilic continuous phase is water, and the preferred hydrophilic component is polyacrylic acid having a weight average molecular weight between about 5,000 and 1,000,000. The solubility parameter index shows that polyacrylic acid is generally swellable in strongly hydrogen bonding solvents but far less swellable in non-hydrogen bonding solvents. Once the hydrophilic portion is determined, then an appropriate hydrophobic portion must be determined, depending upon the composition of the hydrophobic droplet.

The preferred hydrophobic droplet is mineral oil, and therefore an appropriate hydrophobic portion will be readily soluble (or swellable) in mineral oil but substantially insoluble (non-swellable) in water. From a review of solubility parameters, polyacrylic ester is soluble in non-hydrogen bonding solvents and significantly less soluble in strongly hydrogen bonding solvents. Indeed, the preferred emulsifying agent is a polymer comprising a polyacrylic acid portion and a poly acrylic ester portion. The preferred emulsifying agent has a weight ratio of hydrophilic portion to hydrophobic portion of about 3-1000 :1, more preferably about 5-100:1. However, ordinary skill and experimentation may be necessary for any particular embodiment of the present invention to determine the optimal ratio of hydrophobic portion to hydrophilic portion.

Other hydrophobic portions and hydrophilic portions are possible, using the solubility parameter method as a guide. The "solubility parameter" method is a procedure for predicting solubilities of amorphous polymers and, with less justification, crystalline polymers. The value is based upon the free energy change for mixing two pure substances at temperature T, i.e., $\Delta G_{mix} = \Delta H_{mix} - T \Delta S_{mix}$. The solubility parameter method predicts the enthalpy of mixing according to the cohesive energy density, i.e., $E^{coh}$ per unit volume V required to separate completely the molecules in a system (for volatile liquids $E^{coh}$ is usually taken as the energy of vaporization). The solubility parameter is the square root of the cohesive energy density:

$$\delta = (E^{coh}/V)^{\frac{1}{2}} = (E^\circ_{vap}/V)^{\frac{1}{2}} = (H^\circ_{vap} - RT)/V^\circ{}^{\frac{1}{2}}$$

In this equation, $E^\circ_{vap}$ and $H^\circ_{vap}$ are the molar energy and enthalpy of vaporization. A connection between $\Delta H_{mix}$ and the solubility parameters of polymer and solvent is made by writing the intermolecular energies in terms of the cohesive energies for pure solvent and undiluted polymer, and therefore:

$$\Delta H_{mix} = n_1 V^\circ{}_{1\phi 2}(\delta_1 - \delta_2)^2$$

wherein $V^\circ_1$ is the molar volume of the solvent

system, $\phi_2$ is the molar volume fraction of the solute (polymer or monomer system). This equation shows that when the values of the solubility parameters of amorphous polymer and solvent approach one another, $\Delta H_{mix}$ should decrease toward zero. Since the free energy of the mixture decreases with $\Delta H_{mix}$, systems that mix with small heats are expected to have complete miscibility. In applying solubility parameters, miscibility is predicted when $\delta_1$ and $\delta_2$ are within 2 or 3

$$\frac{J^{\frac{1}{2}}}{cm^{3/2}}$$

of one another.

The solubility parameters of most solvents and monomers can be determined from their molar energies or enthalpies of vaporization. High molecular weight polymers however typically cannot be vaporized (they will typically decompose with heating rather than vaporize). Consequently, the solubility parameter for polymers typically must be determined indirectly. Several methods have been employed, most of which use a series of potential solvents with known solubility parameters. In one method, the solubility parameter of the macromolecule is taken as the midpoint of the range of the solubility parameters of those liquids that completely dissolve the polymer. If the polymer is not completely miscible, $\delta_2$ (the solubility parameter of the polymer) is approximated by equating it to the solubility parameter of $\delta_1$ (the solubility parameter of the solvent) of the liquid in which the polymer has the greatest solubility. In another method, the swelling of a lightly cross-linked polymer is measured in the various liquids. The greatest swelling should be found when $\delta_1 \approx \delta_2$. It is also possible to estimate solubility parameters by summing group contributions. The solubility parameters for a large number of polymers and solvents have been compiled, and a rather comprehensive listing can be found in the POLYMER HANDBOOK.

The solubility parameter value for any particular polymer will generally be given as a range according to the solvent system used. The solvent system is generally defined as either strongly, moderately or weakly hydrogen bonding. For each of these solvent types, a solubility parameter range is given for any particular polymer.

If the emulsifying agent requires some fine tuning, a cross-linking agent can be added to diminish the swellability and suspendability of the hydrophilic portion. In this way, the emulsifying agent will be more inclined to move into the boundary layer. Alternatively, other comonomers can be added or the Ph adjusted to alter hydrophilicity. Sometimes the temperature can be adjusted to fine tune the system. Ordinary skill and experimentation may be necessary to find the optimal hydrophobic portion, hydrophilic portion, crosslinker system, Ph and/or temperature range for any particular embodiment of the present invention. The most preferred anionic emulsifying agent is a polyacrylic acid having a poly acrylic ester pendent group wherein the weight ration of polyacrylic acid to poly acrylic ester is about 10,000:1.

Cationic Surfactant Component

Any cationic surfactant can be used in the present invention. The preferred cationic surfactants are dialkyquaternary cationic surfactants. The amount of cationic surfactant is preferably about .01 to about 2% by weight based upon the total weight of the emulsion, although up to about 5% can sometimes be obtained, depending on the particular embodiment of the present invention.

The emulsions of the present invention are useful in conditioning skin and hair. They are also useful in treating wool fibers, laundry fabric softeners, automotive waxes and other applications where dewetting is required by application of aqueous emulsions. Also, the bactericidal properties of cationic surfactants make this technology applicable in antiseptic creams, lotions, dandruff remedies, deodorants, antiperspirants, and oral preparations.

Contact Angle:

Cationic surfactants are effective in treating skin or hair due to head-down, tail-up monolayer adsorption of the surfactant on the hair or skin substrate. This configuration renders the substrate surface hydrophobic and provides advantageous skin or hair "feel" or can be advantageous in altering the substrate from having a hydrophobic surface to having a hydrophilic surface, thereby perhaps enhancing bonding between an organic coating or adhesive and an otherwise polar substrate. The degree of adsorption by the cationic surfactant can be determined by measuring the contact angle of water on a substrate treated with the surfactant. Simply stated, contact angle is a measurement that determines the wettability of water on a substrate.

Examples

A contact angle of zero indicates that the water wets the substrate, i.e., the surface is substantially hydrophilic. A contact angle of 180° means the surface is completely dewetted, i.e., the surface is substantially hydrophobic. Contact angles of water

on glass slides was determined wherein the glass slide was pretreated with one of the following emulsions:

a) emulsions of the present invention comprising: a polyacrylic acid/polyacrylic ester emulsifying agent having a weight average molecular weight of about 500,000 and a weight ratio of polyacrylic acid to polyacrylic ester of about 20:1 (hereafter referred to as "POLYACRYLIC SURFACTANT"); and dialkylquaternium cationic surfactant in varying amount (Emulsions 1-5);

b) commercial cationic emulsions (Eversoft® and Soft-Sense®) having cationic surfactant concentration greater than about 5% by weight (Emulsions 7 and 8); or

c) an aqueous emulsion comprising about 10 weight percent dimethyldistearyl ammonium chloride (emulsion of cationic surfactant alone) (Emulsion 6).

The emulsion examples of the present invention differed in the concentration of dialkylquaternium cation and also POLYACRYLIC SURFACTANT. Emulsions 1 and 2 contained 0.4 wt. percent POLYACRYLIC SURFACTANT with 0.05 and 0.10 percent cationic surfactant respectively. Emulsions 3,4 and 5 contain 0.5 wt. percent POLYACRYLIC SURFACTANT with 0.05, 0.10 and 0.25 wt. percent surfactant respectively.

The contact angles of the cationic emulsion (Emulsion 6) and the commercial emulsions (Emulsions 7 and 8) were about 60-65 degrees. The contact angles of Emulsions 1-5 were 55, 68, 40, 60 and 38. Consequently, a POLYACRYLIC SURFACTANT concentration of about .4 and a cationic surfactant concentration of about .1 is optimal in the preferred embodiment, although lower and higher concentrations also provide hydrophobic surfaces to varying degrees and might be advantageous, depending upon desired hydrophobicity of the substrate.

## Polymerization

The modified polymers are preferably made by polymerization in an inert diluent having some solubilizing action on one or more of the monomeric ingredients but substantially none on the resultant polymer. Polymerization in mass may be employed but is not preferred because of the difficulty in working up the solid polymeric masses obtained. Polymerization in an aqueous medium containing a water-soluble free radical catalyst peroxygen is useful, the product being obtained either as a granular precipitate or as a highly swollen gel, either of which may be used directly or are easily further sub-divided and dried.

Polymerization in an organic liquid which is a solvent for the monomers but a non-solvent for the polymer, or in a mixture of such solvents, in the presence of a solvent-soluble catalyst, is most preferred because the product is usually obtained as a very fine, friable and often fluffy precipitate which, after solvent removal, seldom requires grinding or other treatment before use. Suitable solvents for the latter method include benzene, xylene, tetralin, hexane, heptane, carbon tetrachloride, methyl chloride, ethyl chloride, bromo trichloro methane, ethyl acetate, dimethyl carbonate, diethyl carbonate, ethylene dichloride, and mixtures of these and other solvents.

Polymerization can also be carried out in an aqueous medium of a soluble nonredox multivalent inorganic salt. The acid is too soluble in plain water, therefore, the inorganic salt is added to insolubilize the acid. In this manner, another phase is introduced and the acid is polymerized in a suspension rather than in solution.

The aqueous medium can be a concentrated solution of the salt or it can be a salt slurry of the salt. The difference between the two is considerable. Whereas a concentrated solution of magnesium sulfate salt at reaction temperature is composed of about 2.5 weight parts of the salt per single weight part of water, a slurry of the salt is composed of about 20 weight parts of the salt per single weight part of water. The use of a concentrated salt solution as the reaction medium is preferred.

Although magnesium sulfate is the preferred salt, other organic salts or hydrates thereof can be used, including the nonredox multivalent ion salts such as potassium sulfate, calcium chloride, secondary sodium phosphate and salts employing combinations of anions and cations such as aluminum, barium, beryllium, cadmium, calcium, chloride, chromium, cobalt, lead, magnesium, manganese, molybdate, nickel, selenate, strontium, sulfate, tin tungsten, zinc, and the like.

Success of this polymerization method depends on the fact that the polymerization reaction takes place in discrete and separate oil-in-water droplets. Therefore, water solubility of the inorganic salt employed should be at least about one-half molar in order to salt out the monomer and the formed water-soluble polymer. Moreover, the readily soluble salts can be readily washed out of the finished polymer.

Polymerization in the diluent medium is carried out in the presence of a free radical catalyst in a closed vessel in an inert atmosphere and under autogenous pressure or artificially-induced pressure or in an open vessel under reflux at atmospheric pressure. Temperature of the polymerization may be varied from 0° to 100°C depending to

a large degree on the molecular weight desired in the polymer. Polymerization under reflux at 50° to 90°C under atmospheric pressure using a free radical catalyst is generally effective in bringing a polymer yield of 75% to 100% in less than 10 hours.

Suitable polymerization catalysts include peroxygen compounds such as sodium, potassium and ammonium persulfates, caprylyl peroxide, benzoyl peroxide, hydrogen peroxide, pelargonyl peroxide, cumene hydroperoxides, tertiary butyl diperphthalate, tertiary butyl perbenzoate, sodium peracetate, sodium percarbonate, and the like, as well as azo diisobutyryl nitrile, hereinafter referred to as azoisobutyronitrile. Other catalysts utilizable are the so-called "redox" type of catalysts and the heavy-metal activated catalyst systems.

These amphiphilic polymer emulsifiers of the present invention are preferably made by polymerization in an inert diluent having some solubilizing action on one or more of the monomeric ingredients but substantially none on the resultant polymer. Polymerization in mass may be employed but is not preferred because of the difficulty in processing the solid polymeric masses obtained. Polymerization in an aqueous medium containing a water-soluble free radical catalyst peroxygen can be used wherein the product is a highly swollen gel.

Polymerization in an organic liquid which is a solvent for the monomers but a non-solvent for the polymer, or in a mixture of such solvents, in the presence of a solvent-soluble catalyst, is most preferred. Suitable solvents for the preferred embodiment include benzene, xylene, tetralin, hexane, heptane, carbon tetrachloride, methyl chloride, ethyl chloride, bromo trichloro methane, ethyl acetate, dimethyl carbonate, diethyl carbonate, ethylene dichloride, and mixtures of these and other solvents.

Polymerization can also be carried out in an aqueous medium of a soluble nonredox multivalent inorganic salt. The acid is too soluble in plain water, therefore, the inorganic salt is added to insolubilize the acid. In this manner, another phase is introduced and the acid is polymerized in an suspension rather than in solution.

The aqueous medium can be a concentrated solution of the salt or it can be a salt slurry of the salt. The difference between the two is considerable. Whereas a concentrated solution of magnesium sulfate salt at reaction temperature is composed of about 2.5 weight parts of the salt per single weight part of water, a slurry of the salt is composed of about 20 weight parts of the salt per single weight part of water. The use of a concentrated salt solution as the reaction medium is preferred.

Although magnesium sulfate is the preferred salt, other organic salts or hydrates thereof can be used, including the nonredox multivalent ion salts such as potassium sulfate, calcium chloride, secondary sodium phosphate and salts employing combinations of anions and cations such as aluminum, barium, beryllium, cadmium, calcium, chloride, chromium, cobalt, lead, magnesium, manganese, molybdate, nickel, selenate, strontium, sulfate, tin tungsten, zinc, and the like.

Success of this polymerization method depends on the fact that the polymerization reaction takes place in discrete and separate oil-in-water droplets. Therefore, water solubility of the inorganic salt employed should be at least about one-half molar in order to salt out the monomer and the formed water-soluble polymer. Moreover, the readily soluble salts can be readily washed out of the finished polymer.

Polymerization in the diluent medium is preferably carried out in the presence of a free radical catalyst in a closed vessel in an inert atmosphere and under autogenous pressure or artificially-induced pressure or in an open vessel under reflux at atmospheric pressure. Temperature of the polymerization may be varied from 0° to 100°C depending to a large degree on the molecular weight desired in the polymer. Polymerization under reflux at 50° to 90°C under atmospheric pressure using a free radical catalyst is generally effective in bringing a polymer yield of 75% to 100% in less than 10 hours.

Suitable polymerization catalysts include peroxygen compounds such as sodium, potassium and ammonium persulfates, caprylyl peroxide, benzoyl peroxide, hydrogen peroxide, pelargonyl peroxide, cumene hydroperoxides, tertiary butyl diperphthalate, tertiary butyl perbenzoate, sodium peracetate, sodium percarbonate, and the like, as well as azo diisobutyryl nitrile, hereinafter referred to as azoisobutyronitrile. Other catalysts utilizable are the so-called "redox" type of catalysts and the heavy-metal activate catalyst systems.

These modified polymers generally do not attain their maximum properties in water until converted to a partial alkali, ammonium or amine salt. The neutralizing agent is preferably a monovalent alkali such as sodium, potassium, lithium or ammonium hydroxide or the carbonates and bicarbonates thereof, or mixtures of the same, and also amine bases having not more than one primary or secondary amino group.

Conventional oil-in-water emulsions have particle size of less than 10 microns, preferably 0.1-5 microns. Surprisingly, the oil-in-water emulsions of the present invention can be prepared with the modified polymer having a much larger particle size averaging about 50 microns or more.

The modified polymer emulsifiers of the present invention function as primary emulsifiers or surfactants whereas hydrophilic polymers devoid of a hydrophobic portion do not possess this property. Although polyacrylic acids which are partially neutralized with long chain alkylamines may be capable of behaving as primary emulsifiers, such polyacrylic acids, when used to prepare the emulsion, yield emulsions which are stable only at high Ph values above about 6 whereas the emulsions of the present invention prepared with the modified polymer containing long chain acrylate monomers are stable at low Ph of about 3 to 6.

While several embodiments of the invention have been shown and described, various adaptations and modifications can be made by one skilled in the art without departing from the scope of the invention as defined in the appended claims. For instance, the preferred embodiment of an oil in water emulsion is not critical to the present invention, and the present invention also includes water in oil emulsions wherein water is the discontinuous phase and oil is the continuous phase. In such a system the emulsifying agent would also be ampiphilic, but the predominant portion would be hydrophobic to support the suspended water droplet and the lesser portion would be hydrophilic to anchor the emulsifier to the water droplet. Numerous other embodiments are certainly possible and ordinary skill and experimentation for any particular embodiment, depending upon the intended continuous and discontinuous phase of the emulsion.

## Claims

1. An emulsion composition comprising:
   a hydrophilic continuous phase;
   a hydrophobic discontinuous phase;
   a cationic surfactant having a weight average molecular weight less than about 5000;
   an emulsifying agent having a weight average molecular weight greater than that of the cationic surfactant, said emulsifying agent comprising a hydrophilic portion and a hydrophobic portion, said hydrophilic portion comprising anionic functionality, whereby the weight ratio of the hydrophilic portion to the hydrophobic portion is about 3-1000:1.

2. The emulsion of Claim 1 wherein the continuous phase comprises water and wherein the weight percent of cationic surfactant based upon the total weight of the emulsion is about .001 to about 5 and the weight percentage of the emulsifying agent based upon the total weight of the emulsion is about .001 to about 5.

3. The emulsion of Claim 2 wherein the hydrophobic discontinuous phase comprises hydrophobic droplets having a diameter greater than about 10 microns.

4. The emulsion of Claim 3 wherein the droplets have a diameter greater than about 25 microns.

5. The emulsion of Claim 4 wherein the droplets have a diameter greater than about 50 microns.

6. The composition of Claim 1 wherein greater than about 50% of the cationic surfactant attaches to a glass substrate to provide a contact angle of greater than about 35 degrees within about 1 minute after the emulsion is applied to the glass substrate.

7. The emulsion of Claim 6 wherein greater than about 80% of the cationic surfactant is attaches to the substrate and the contact angle is greater than about 60 degrees.

8. An emulsifying agent having a weight average molecular weight greater than about 1000, said emulsifying agent comprising a hydrophilic portion and a hydrophobic portion, said hydrophilic portion comprising anionic functionality, whereby the weight ratio of the hydrophilic portion to the hydrophobic portion is about 3 to 1000:1.

9. The emulsifying agent of Claim 8 further comprising an organic solvent residue.

10. The emulsifying agent of Claim 9 wherein the residue is ethyl acetate, benzene, or a halogenated aliphatic.

11. The emulsifying agent of Claim 10 further comprising a water soluble free radical catalyst residue.

12. The emulsifying agent of Claim 11 wherein the hydrophilic portion comprises carboxylic functionality.

13. The emulsifying agent of Claim 12 wherein the hydrophilic portion comprises a monoolefinic acrylic acid or derivative thereof.

14. The emulsifying agent of Claim 13 wherein the hydrophobic portion extends away from the hydrophilic portion.

**15.** The composition of Claim 3 wherein the cationic surfactants is a dialkyquaternary cationic surfactant.

European Patent Office

# EUROPEAN SEARCH REPORT

Application Number

EP 91117109.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A<br><br><br><br>X | EP - A - 0 268 164<br>(THE B.F. GOODRICH CO.)<br>* Claims; page 6, line 16 - page 8, line 24 *<br>-- | 1-7, 15<br><br><br><br>8-14 | A 61 K 7/00 |
| A | US - A - 4 552 755<br>(N.A. RANDEN)<br>* Claims *<br>-- | 1-15 | |
| A | US - A - 3 915 921<br>(R.K. SCHLATZER)<br>* Claims; examples; abstract *<br>---- | 8-14 | |
| | | | TECHNICAL FIELDS SEARCHED (Int. Cl.5) |
| | | | A 61 K 7/00 |

The present search report has been drawn up for all claims

| Place of search | Date of completion of the search | Examiner |
|---|---|---|
| VIENNA | 23-12-1991 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)